# EUROPEAN PATENT APPLICATION

(11) **EP 4 603 036 A1**
(43) Date of publication of application: **20.08.2025**
(21) Application number: 25157257.4
(22) Date of filing: 11.02.2025
(51) Int. Cl.: A61B 17/32

(54) **SURGICAL BUR, SURGICAL SYSTEM, AND METHOD FOR OPERATING THE SURGICAL SYSTEM**

(30) Priority: 16.02.2024 JP 2024022122
(71) Applicant: Nakanishi Inc., Kanuma-shi, Tochigi 322-8666 (JP)
(72) Inventor: IITSUKA, Takamitsu, Kanuma-shi, 3228666 (JP); AIBA, Yoshiki, Kanuma-shi, 3228666 (JP)
(74) Representative: Lewis Silkin LLP

(57) **Abstract**

A surgical bur 10 includes a bar-shaped shaft 11 and a cutting part 13 provided at one axial end of the shaft 11, which rotates around the axis of the shaft 11. The shaft 11 is formed on the outer peripheral surface on the cutting part 13 side in the axial direction and includes a liquid flow generation part 15 having at least one of a convex part projecting outward in the radial direction or a concave part recessed inward in the radial direction.

## Description

### TECHNICAL FIELD

The present invention relates to a surgical bur, a surgical system, and a method for operating the surgical system.

### BACKGROUND ART

Conventionally, one of the medical instruments used in surgery, known as surgical burs, is used with bone surgery instruments. Exemplary surgical burs are disclosed in related art documents such as JP 2013-502943 A, JP 2016-527003 A and JP 2019-531140 A. A surgical bur has a shaft and a cutting part provided at the tip of the shaft. The cutting part has blades that cut tissues such as bones. The shaft is fixed to the drive shaft of a handpiece or similar device.

During surgery, by operating the drive shaft to rotate the surgical bur and pressing the cutting part against the tissue to be removed (target tissue), it is removed by the rotating blades. Around the area to be removed, the surrounding area is filled with perfusion fluid (disinfected water, saline solution, irrigation, etc.) supplied externally, and the surgical bur rotates in the perfusion fluid to remove the target tissue.

Such surgical burs are used in various surgeries such as orthopedic surgery, neurosurgery, spinal surgery, and ENT surgery, or in procedures for selectively removing parts of tissues.

### SUMMARY OF INVENTION

However, in the surgical burs disclosed in the documents listed above, there is an issue where cut tissue from the target removal position floats around, obstructing the surgeon's view during the cutting operation. Although a certain amount of perfusion fluid is continuously supplied around the removal target position, it may be insufficient to improve the view of the surgeon at the removal target position.

The present invention aims to solve the above problem by ensuring the surgeon's view around the removal target position during the tissue removal work using a surgical bur, thereby improving the safety, accuracy, and speed of the work.

The present invention provides at least the following configuration:
(1) A surgical bur including a bar-shaped shaft and a cutting part provided at one axial end of the shaft, which rotates around the axis of the shaft,
   wherein the shaft is formed on the outer peripheral surface on the cutting part side in the axial direction and includes a liquid flow generation part having at least one of a convex part projecting outward in the radial direction or a concave part recessed inward in the radial direction.
(2) A surgical system including:
   the surgical bur described in (1);
   a handpiece having a long tubular support that exposes the cutting part and the liquid flow generation part at the tip and rotationally supports the shaft;
   a rotation control device that rotationally drives the shaft of the surgical bur supported by the tubular support; and
   a perfusion fluid device that supplies perfusion fluid towards the cutting part and the liquid flow generation part of the surgical bur.
(3) A method for operating the surgical system described in (2),
   wherein the tip of the tubular support of the handpiece supporting the surgical bur is inserted into the affected area, and the perfusion fluid device supplies perfusion fluid to the cutting area by the surgical bur, and
   wherein while the cutting part and the liquid flow generation part are placed in the liquid perfusion fluid accumulated in the affected area, the rotation control device rotationally drives the surgical bur, generating a flow of the perfusion fluid by the liquid flow generation part.

According to the present invention, it is possible to ensure the surgeon's view around the removal target position during the tissue removal work using the surgical bur, thereby improving the safety, accuracy, and speed of the work.

### BRIEF DESCRIPTION OF DRAWINGS

FIG. 1 is a schematic diagram showing the main configuration of a surgical system.
FIG. 2 is an overall configuration diagram of the surgical bur according to a first embodiment.
FIG. 3 is a partially enlarged view showing the tip of the surgical bur attached to the handpiece attachment.
FIG. 4 is an explanatory diagram schematically showing the flow of perfusion fluid due to the rotation of the surgical bur.
FIG. 5 is an explanatory diagram schematically showing the flow of perfusion fluid when the surgical bur is rotated in the opposite direction to that shown in FIG. 4.
FIG. 6 is an explanatory diagram schematically showing the state of the surgery using the surgical bur.
FIG. 7 is a partially enlarged view showing the tip of the surgical bur according to a second embodiment attached to the handpiece attachment.
FIG. 8 is a front view of the cutting part seen from the tip of the surgical bur.
FIG. 9 is a partial cross-sectional view cut along the axial direction of the liquid flow generation part.
FIG. 10 is a partial cross-sectional view cut along the axial direction of the liquid flow generation part serving as a cutting blade.
FIG. 11 is a perspective view showing the tip of the surgical bur according to a first modification of the liquid flow generation part.
FIG. 12 is a perspective view showing the tip of the surgical bur according to a second modification of the liquid flow generation part.
FIG. 13 is a cross-sectional view taken along the line XIII-XIII shown in FIG. 12.
FIG. 14 is a perspective view showing the tip of the surgical bur according to a third modification of the liquid flow generation part.

### DESCRIPTION OF EMBODIMENTS

Hereinafter, the surgical bur and surgical system in which the surgical bur is used according to the present invention will be described in detail with reference to the drawings. The surgical system exemplified here is configured for use in spinal surgery, but the application of the present invention is not limited thereto.

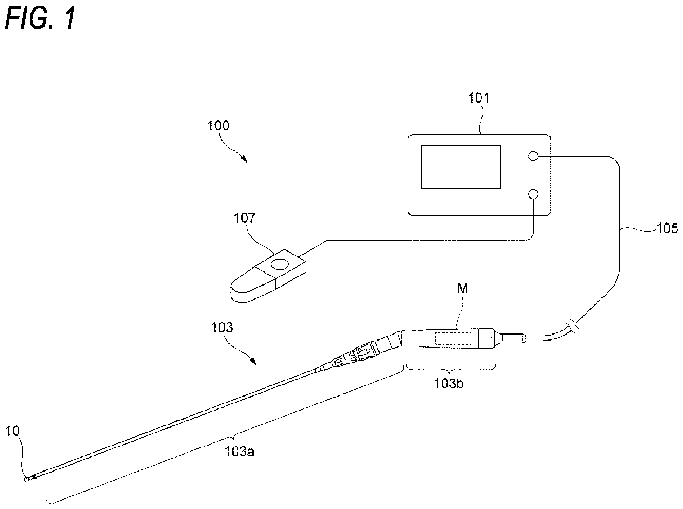

FIG. 1 is a schematic diagram showing the main configuration of a surgical system 100. The surgical system 100 includes a controller 101, a handpiece 103, a connection cable 105 connecting the handpiece 103 to the controller 101, and a foot switch 107.

The handpiece 103 includes an attachment 103a and a grip 103b. The attachment 103a at the tip of the handpiece 103 is a long tubular support that supports the surgical bur 10 detachably. The grip 103b of the handpiece 103 is provided with a power source M such as an air motor or electric motor (electric motor) that rotationally drives the surgical bur 10. The power source M is rotationally controlled by the foot switch 107 connected to the controller 101. The controller 101 controls various operations such as the rotational speed and direction of the surgical bur 10 by the handpiece 103. This driving control is realized by the operation of a computer that includes a processor, memory, and storage. The power source M and the controller 101 described above function as a rotation control device that rotationally drives the shaft 11 of the surgical bur 10 supported by the attachment 103a.

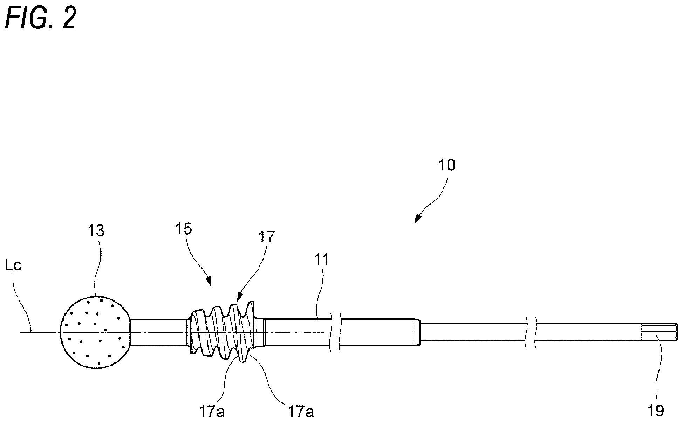

FIG. 2 is an overall configuration diagram of the surgical bur 10 according to a first embodiment. The surgical bur 10 includes a bar-shaped shaft 11 that extends along the rotation axis Lc and a ball-shaped cutting part 13 provided at one end of the shaft 11. Diamond abrasive grains or the like (not shown) are electrodeposited on the surface of the cutting part 13. Alternatively, a cutting blade may be formed instead of the abrasive grains mentioned above. The "ball shape" herein means that when the surgical bur 10 is rotated around the rotation axis Lc, the surface shape formed by enveloping the abrasive grains or cutting edge formed on the cutting part 13 is a sphere or a rotational ellipsoid centered on the rotation axis Lc.

The shaft 11 includes a liquid flow generation part 15 formed on the outer peripheral surface on the cutting part 13 side in the axial direction. The liquid flow generation part 15 of this configuration is a spiral protrusion 17 formed spirally around the outer circumference of the shaft 11, centering on the rotation axis Lc (hereinafter also simply referred to as the axis), and protruding in the radial direction. The spiral protrusion 17 shown here is a single protrusion with a pair of wall surfaces 17a extending circumferentially on both sides in the axial direction. The position of the liquid flow generation part 15 on the shaft 11 is set according to the size and application of the surgical bur 10. For example, the distance S from the base end 13a of the cutting part 13 to the axial end of the liquid flow generation part 15 may be set within the range of ±20% of the maximum outer diameter dimension Dp of the cutting part 13.

The surgical bur 10 is formed of a hard material such as stainless steel or a hard alloy (tungsten carbide). The base end on the side opposite to the cutting part 13 of the shaft 11 has a connecting part 19 fixed to the drive shaft (not shown) of the power source M of the handpiece 103.

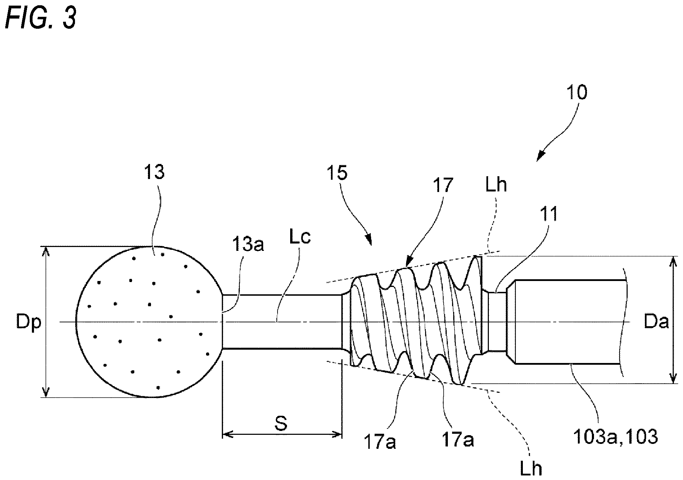

FIG. 3 is a partially enlarged view showing the tip of the surgical bur 10 attached to the attachment 103a of the handpiece 103. The surgical bur 10 exposes the cutting part 13 and the liquid flow generation part 15 from the tip of the attachment (tubular support) 103a, and the shaft 11 is rotationally supported by the handpiece 103. The surgical bur 10 fixed to the rotation shaft is rotated around the rotation axis Lc by the rotational driving from the handpiece 103.

In this surgical system 100, the surgeon operates the foot switch 107 while holding the handpiece 103 shown in FIG. 1 to rotate the surgical bur 10 at high speed. Then, when the surgeon moves the handpiece 103 while pressing the cutting part 13 of the surgical bur 10 against the desired part, the rotating cutting part 13 can cut that part. During surgery, perfusion fluid W such as saline or sterilized water is supplied to the cutting area (also referred to as the affected area), and the liquid flow generation part 15 generates a flow in the perfusion fluid W present around the affected area due to the rotation of the surgical bur 10. This flow of perfusion fluid W improves the surgeon's view of the affected area.

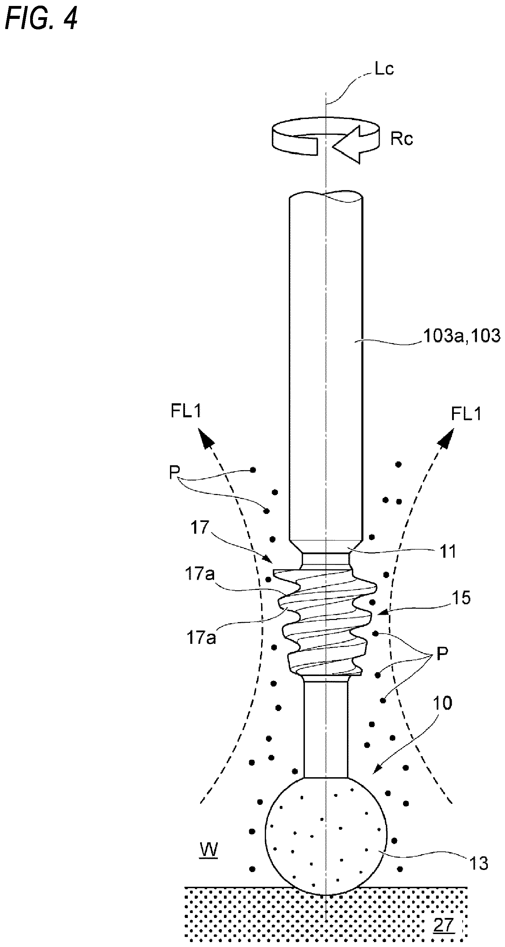

FIG. 4 is an explanatory diagram schematically showing the flow of perfusion fluid due to the rotation of the surgical bur 10. As shown in FIG. 4, the surgical bur 10 rotates around the rotation axis Lc in the direction Rc (clockwise when viewed from above the rotation axis Lc). Since the spiral protrusion 17 of the liquid flow generation part 15 is a right-handed spiral (same as right-hand threads), the wall surface 17a of the spiral protrusion 17 advances from the cutting part 13 side to the handpiece 103 side along the shaft 11, producing a feed motion. This feed motion of the spiral protrusion 17 generates a flow indicated by arrow FL1 in the perfusion fluid W around the spiral protrusion 17. The perfusion fluid W, flowing in the direction of arrow FL1, eliminates the perfusion fluid W containing tissue fragments P cut by the cutting part 13 from the vicinity of the cutting part 13. Consequently, new perfusion fluid W with high transparency that does not contain tissue fragments P flows into the vicinity of the cutting part 13, maintaining a good view of the vicinity of the cutting part 13. In other words, the view around the cutting area during the cutting operation is kept clear, improving the surgeon's workability.

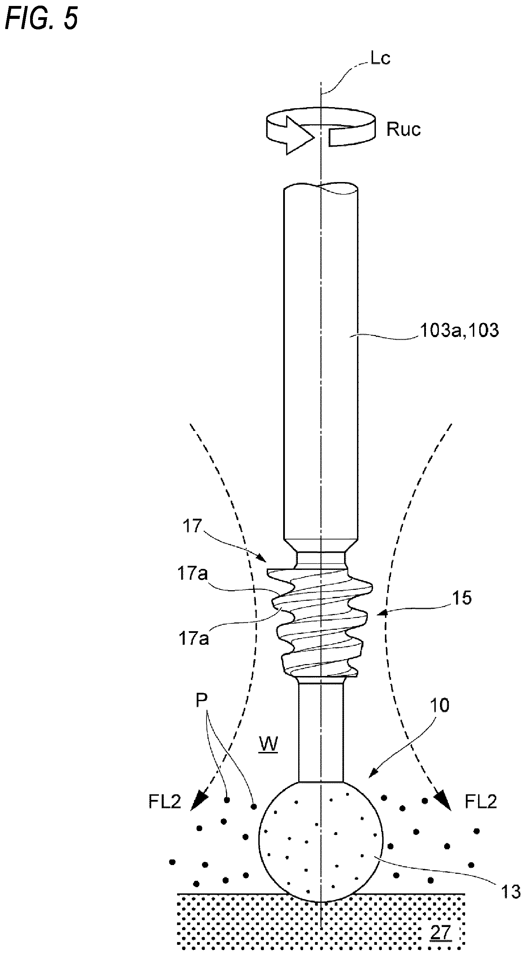

FIG. 5 is an explanatory diagram schematically showing the flow of perfusion fluid W when the surgical bur 10 is rotated in the opposite direction to that shown in FIG. 4. As shown in FIG. 5, the surgical bur 10 rotates around the rotation axis Lc in the direction Ruc (counterclockwise when viewed from above the rotation axis Lc). The wall surface 17a of the spiral protrusion 17 of the liquid flow generation part 15 advances from the handpiece 103 side to the cutting part 13 side along the shaft 11, producing a feed motion. This feed motion of the spiral protrusion 17 generates a flow indicated by arrow FL2 in the perfusion fluid W around the spiral protrusion 17, eliminating the perfusion fluid W containing tissue fragments P cut by the cutting part 13 from the vicinity of the cutting part 13.

The direction of the perfusion fluid W flow can be in either directions shown by arrows FL1 and FL2, but when a circulation path is formed for the perfusion fluid W, it is preferable to direct the perfusion fluid W towards the discharge direction. The rotation direction of the surgical bur 10 can be set by the controller 101 shown in FIG. 1 and can be appropriately changed by the surgeon according to the situation. The flow rate of the perfusion fluid W can be easily adjusted by increasing or decreasing the rotational speed of the surgical bur 10 using the controller 101.

Additionally, by changing the spiral protrusion 17 from a right-handed spiral to a left-handed spiral, the direction of the perfusion fluid W flow can be reversed. When the outer diameter of the shaft 11 is 1.5 mm, the preferred pitch (the axial advancement per rotation) of the spiral protrusion 17 is, for example, preferably 0.3 mm to 2.5 mm, more preferably 0.5 mm to 1.5 mm. The spiral protrusion 17 is not limited to a single protrusion and may comprise multiple protrusions. The axial length of the liquid flow generation part 15 is preferably 2 mm to 10 mm, more preferably 3 mm to 5 mm.

As shown in FIG. 3, it is preferable that the spiral protrusion 17 has a tapered shape, with the protrusion height in the radial direction being lower closer to the cutting part 13. In other words, the enveloping line Lh of the outer edge of the spiral protrusion 17 becomes shorter in distance from the rotation axis Lc as it approaches the cutting part 13. By making the protrusion height lower closer to the cutting part 13, the view near the cutting part 13 of the surgeon during surgery is less obstructed by the spiral protrusion 17, making it easier to maintain a wider field of view.

Moreover, it is preferable that the maximum outer diameter dimension Dp of the spiral protrusion 17 is smaller than the maximum outer diameter dimension Dc of the cutting part 13. This prevents the surgeon's view from being obstructed by the spiral protrusion 17 during surgery and makes it easier to secure the view near the cutting part 13. Additionally, because the protrusion height of the spiral protrusion 17 is lower than that of the cutting part 13, unnecessary tissue is less likely to be inadvertently damaged by the spiral protrusion 17.

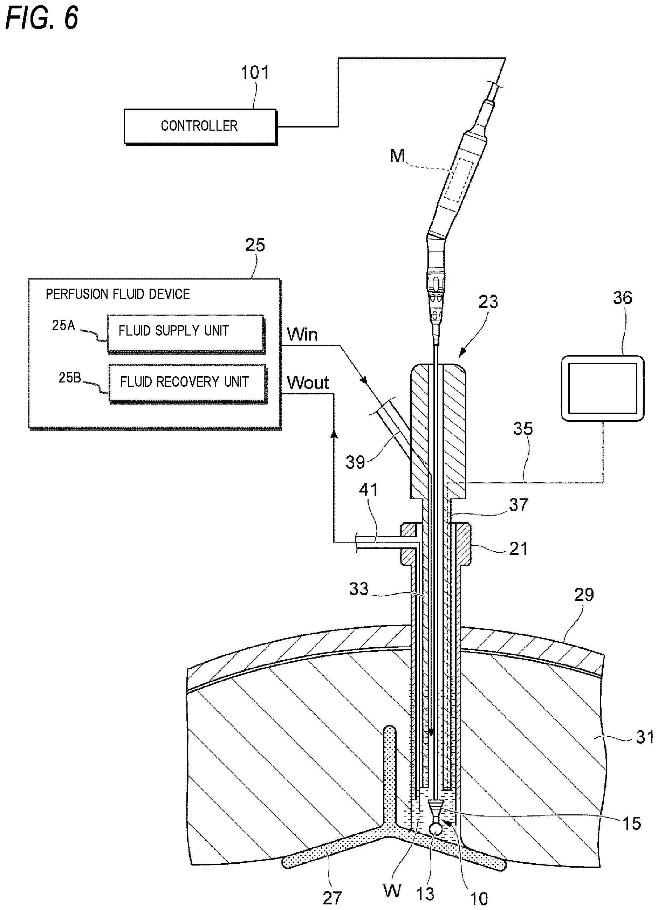

FIG. 6 is an explanatory diagram schematically showing the state of a surgery using the surgical bur 10. The surgical system used includes the previously described components shown in FIG. 1 as well as an additional tubular sheath 21, an endoscopic device 23 (percutaneous spinal endoscope), and a perfusion fluid device 25. Here, an example of cutting a patient's bone 27 is described.

The perfusion fluid device 25 includes a fluid supply unit 25A that delivers perfusion fluid W to the affected area and a fluid recovery unit 25B that sucks and discharges the perfusion fluid W from the affected area. The fluid supply unit 25A and the fluid recovery unit 25B are provided with pumps (not shown) that deliver or recover the perfusion fluid W. In this configuration, the fluid supply unit 25A supplies the perfusion fluid W to the affected area through the endoscopic device 23 inserted inside the sheath 21, and the fluid recovery unit 25B sucks and discharges the perfusion fluid W around the affected area through the inside of the sheath 21.

The endoscopic device 23 has a cylindrical insertion part 33 that is inserted into the affected area, an observation optical system 35 that sends illumination light from the tip of the insertion part 33, illuminates the area around the cutting part 13, and acquires an observation image, a working lumen 37 into which various instruments are inserted, and a supply path 39 that supplies perfusion fluid W to the working lumen 37. The endoscopic device 23 outputs the imaging information of the observation image obtained from the observation optical system 35, as well as various information such as the imaging conditions and imaging position. The configuration may also allow the surgeon to observe the observation image visually obtained from the observation optical system 35. When the configuration outputs imaging information, an image display device such as a monitor connected to the endoscopic device 23 displays the imaging information, enabling the surgeon to easily view the imaging information.

The sheath 21 is provided with a discharge path 41 for discharging the perfusion fluid W inside the sheath 21. The supply path 39 connects to the fluid supply unit 25A of the perfusion fluid device 25, and the discharge path 41 connects to the fluid recovery unit 25B.

First, the patient's skin 29 and muscles 31 are incised, and the sheath 21 is inserted close to the bone 27 planned for cutting. Then, the insertion part 33 of the endoscopic device 23 is inserted inside the sheath 21. After inserting the insertion part 33 of the endoscopic device 23 into the body cavity, the fluid supply unit 25A of the perfusion fluid device 25 supplies the perfusion fluid W to the working lumen 37 through the supply path 39. This supplies perfusion fluid W from the working lumen 37 to the affected area, and the cutting part 13 and the liquid flow generation part 15 are positioned in the liquid perfusion fluid W accumulated in the affected area. Furthermore, the perfusion fluid W around the affected area is sucked and discharged by the fluid recovery unit 25B through the discharge path 41, ensuring that the perfusion fluid W around the affected area is sequentially replaced without stagnation.

Then, the attachment 103a of the handpiece 103, with the surgical bur 10 attached to its tip, is inserted into the working lumen 37 of the endoscopic device 23. The inserted attachment 103a is rotationally driven by the rotation control device consisting of the power source M and the controller 101, enabling bone cutting by the rotating cutting part 13. The condition of the cutting by the cutting part 13 can be visually confirmed by looking at the observation image obtained by illuminating the affected area around the cutting part 13 with illumination light from the observation optical system 35, or by displaying the imaging image on a monitor or other image display device.

As described above, the surgeon can operate the handpiece 103 and perform on-off control of the rotation of the surgical bur 10 using the foot switch 107 shown in FIG. 1 while visually confirming the observation image obtained from the observation optical system 35, cutting the bone with the surgical bur 10.

When cutting the bone, tissue fragments generated during cutting mix with the perfusion fluid, causing the perfusion fluid W to become suspended, making it difficult to see the cutting state in the observation image. However, since the liquid flow generation part 15 provided in the surgical bur 10 generates a flow in the perfusion fluid W due to the rotational drive, the suspended perfusion fluid W near the cutting part is carried away to another area by the flow, being replaced with new perfusion fluid W. As a result, the area around the cutting part 13 is always filled with new perfusion fluid W, making it possible to obtain a clear observation image of the vicinity of the cutting part 13.

The flow of the perfusion fluid W generated by the liquid flow generation part 15 flows towards the base end side opposite to the cutting part 13, as shown in FIG. 4. In this way, when the liquid flow generation part 15 generates a flow of perfusion fluid W along the shaft 11 towards the liquid flow generation part 15, the suspended perfusion fluid W around the cutting part 13 is carried away by the flow and smoothly discharged from the working lumen 37 by suction from the fluid recovery unit 25B.

Additionally, when the flow of the perfusion fluid W is directed towards the cutting part 13, as shown in FIG. 5, new perfusion fluid W supplied by the fluid supply unit 25A can be provided to the cutting part 13. In other words, the liquid flow generation part 15 generates a flow of the perfusion fluid W around the cutting part 13 towards the opposite side of the shaft 11. This ensures that the area around the cutting part 13 is always filled with new perfusion fluid W, allowing for a clear observation image to be obtained.

No matter the direction of the flow of the perfusion fluid W, the perfusion fluid W will not stagnate, quickly cooling the cutting part 13 and the affected area cut by the cutting part 13, suppressing the rise in temperature. Therefore, bone damage caused by heat generated during bone cutting is less likely to occur. Consequently, during the cutting operation of the target tissue, it is possible to stably ensure the surgeon's field of view around the cutting position. Thus, the safety, accuracy, and speed of the work can be improved.

In the above-described surgery, the sheath 21 and the endoscopic device 23 are used, but it may also be possible to supply the perfusion fluid W to the incised affected area and perform the cutting with the surgical bur 10 by rotating the surgical bur 10 while operating the handpiece 103. In that case, as well, rotating the cutting part 13 and the liquid flow generation part 15 of the surgical bur 10 in the perfusion fluid W allows the condition of the cutting operation to be clearly visible.

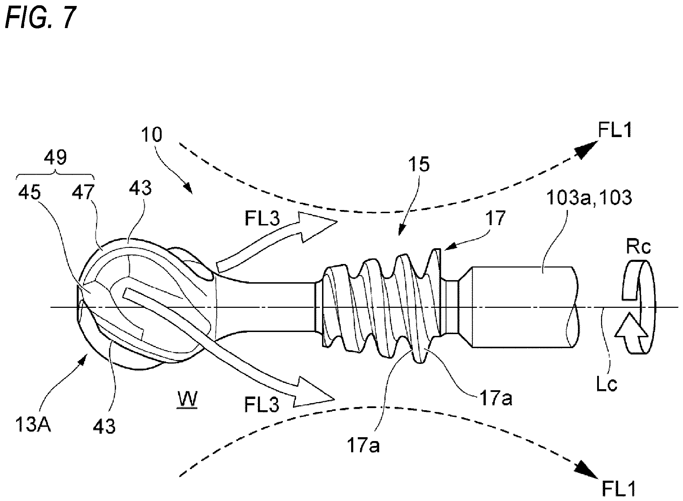

FIG. 7 is a partially enlarged view showing the tip of the surgical bur 10 of a second embodiment attached to the attachment 103a of the handpiece 103. In this configuration, the cutting part 13A of the surgical bur 10 extends from the axial tip to the axial base and includes multiple rows of cutting blades 43 formed in rotationally symmetrical positions around the rotation axis Lc. Between the multiple rows of cutting blades 43, there are rake faces 45 and relief faces 47 of the cutting blades 43 adjacent circumferentially, with recessed grooves 49 provided therebetween.

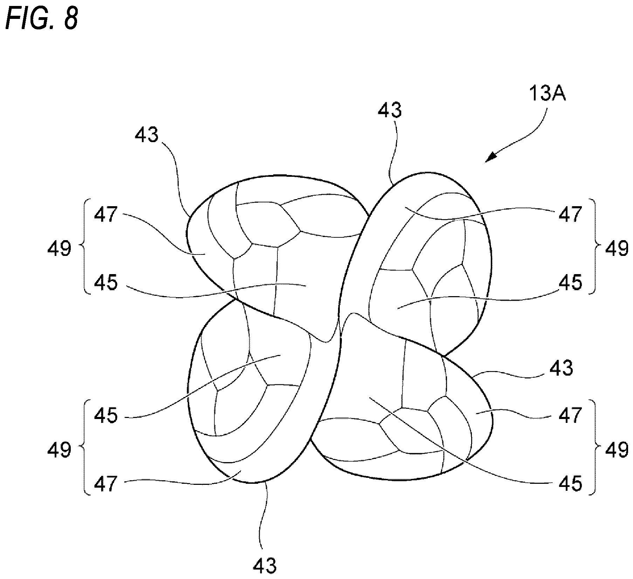

FIG. 8 is a front view of the cutting part 13A seen from the tip of the surgical bur 10. The cutting part 13A of this configuration has four cutting blades 43, with cutting blades 43 and grooves 49 alternately arranged circumferentially around the cutting part 13A. As shown in FIG. 7, the multiple rows of cutting blades 43 are formed spirally along the axial direction, and consequently, the grooves 49 are also formed spirally. Therefore, with the rotation Rc of the surgical bur 10, the perfusion fluid W in the grooves 49 generates a spiral flow indicated by arrow FL3.

The flow FL3 of the perfusion fluid W from the cutting part 13A is in the same direction as the flow FL1 caused by the spiral protrusion 17 in the liquid flow generation part 15, promoting flow FL1. As a result, the perfusion fluid W containing tissue fragments P cut by the cutting part 13A is efficiently removed from the vicinity of the cutting part 13A, and new transparent perfusion fluid W without tissue fragments P flows into the vicinity of the cutting part 13A. Consequently, the view around the cutting part 13A becomes more favorable, ensuring a good view of the removal position during the cutting operation.

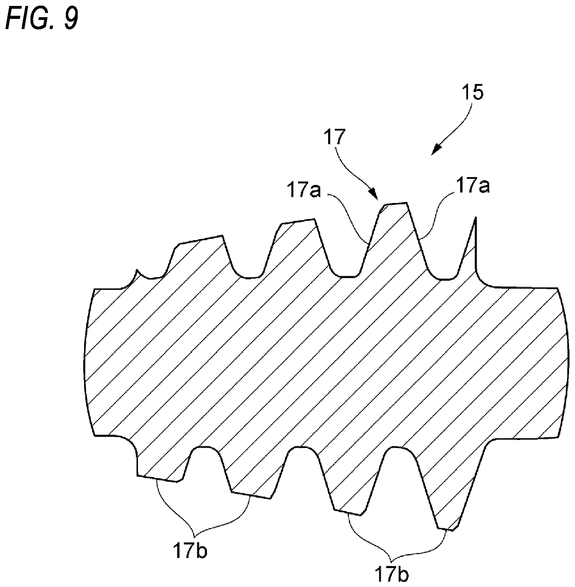

FIG. 9 is a partial cross-sectional view cut along the axial direction of the liquid flow generation part 15. The spiral protrusion 17 of the liquid flow generation part 15 is formed with a flat top surface 17b with a lower radial height at the protruding tip, removing sharp pointed sections. By removing pointed sections from the top surface 17b of the spiral protrusion 17, contact with tissue during the surgery can prevent damaging the contacted tissue.

On the other hand, the shape of the top of the spiral protrusion 17 can be made sharp, allowing the spiral protrusion 17 to function as a cutting blade.

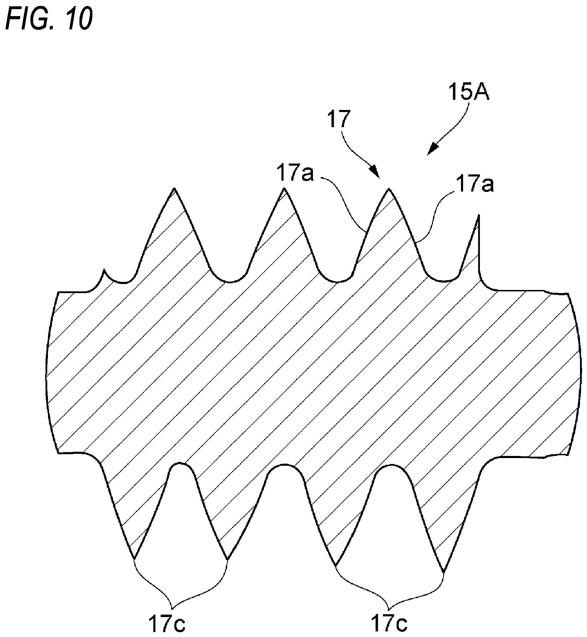

FIG. 10 is a partial cross-sectional view cut along the axial direction of the liquid flow generation part 15A serving as a cutting blade. The spiral protrusion 17A formed in the liquid flow generation part 15A has a sharply pointed tip 17c. The pointed tip 17c can contribute to cutting tissues. Therefore, by pressing both the cutting part 13 and the pointed tip 17c of the spiral protrusion 17A against the tissue to be cut during surgery, a wide range of tissues can be efficiently removed.

Next, modifications of the liquid flow generation part will be described. The liquid flow generation part 15 is not limited to the above-described spiral protrusion 17 and can have any shape that can create a flow of perfusion fluid. For example, a shape that has an effect of stirring the perfusion fluid may be used. Each modification of the liquid flow generation part shown below is simplified in shape, but can include parts of the shapes and configurations exemplified in the liquid flow generation part.

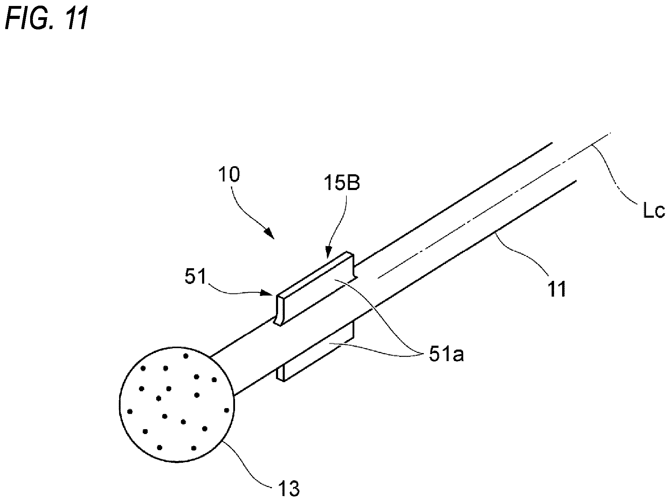

FIG. 11 is a perspective view showing the tip of the surgical bur 10 according to a first modification of the liquid flow generation part 15B. This liquid flow generation part 15B includes a flat plate 51 as a convex part protruding outward in the radial direction from the outer peripheral surface of the shaft 11 and extending along the rotation axis Lc, and a continuous wall surface 51a. At least one flat plate 51 is provided circumferentially on the shaft 11, preferably multiple ones in rotation symmetric positions. With this configuration, as the surgical bur 10 rotates, the wall surface 51a of the flat plate 51 stirs the surrounding perfusion fluid, generating a flow in the perfusion fluid.

Although not shown, the entire flat plate 51 or a part of it may be inclined from the rotation axis Lc to one side in the circumferential direction of the shaft 11. This means that the wall surface 51 a of the flat plate 51 may be inclined at a predetermined angle in the same direction like a screw. In this case, as the surgical bur 10 rotates, a flow will be generated along the shaft 11 in the perfusion fluid W. The flat plate 51 does not have to be inclined entirely; for example, only a part of the axial direction of the flat plate 51 may be inclined.

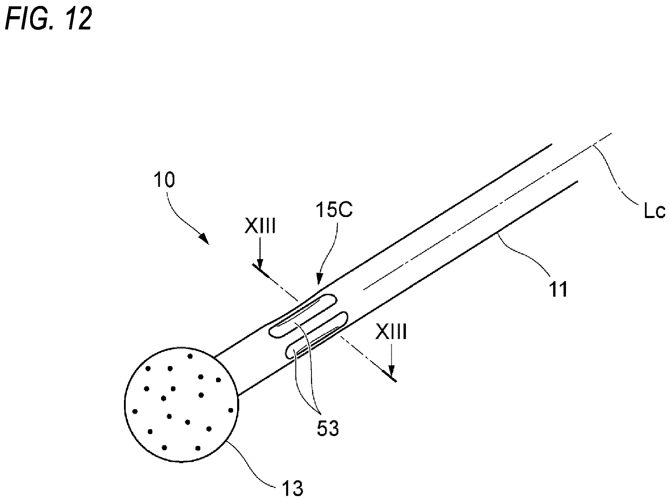

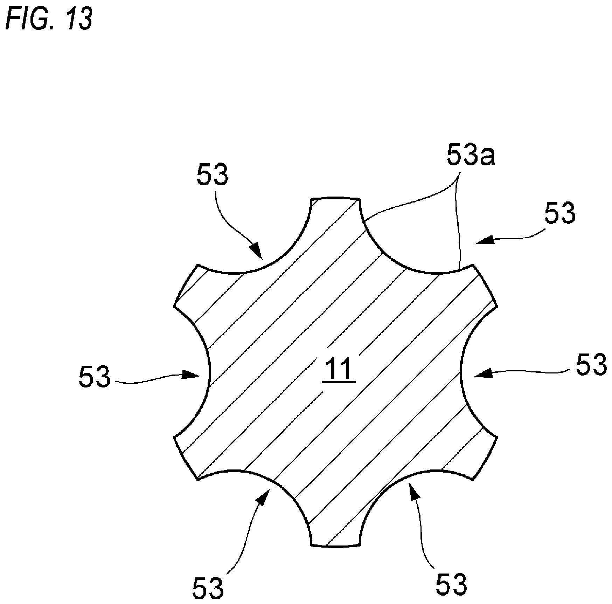

FIG. 12 is a perspective view showing the tip of the surgical bur 10 according to a second modification of the liquid flow generation part 15C. FIG. 13 is a cross-sectional view along the line XIII-XIII shown in FIG. 12. This liquid flow generation part 15C includes a recessed groove 53 recessed inward in the radial direction from the outer peripheral surface of the shaft 11. The recessed groove 53 is provided in at least one place in the circumferential direction of the shaft 11, preferably multiple places in rotation symmetric positions. The recessed groove 53 has an inner wall surface 53a along the shaft 11. As the surgical bur 10 rotates, the inner wall surface 53a stirs the perfusion fluid W, generating a flow in the perfusion fluid W.

Although not shown, the entire recessed groove 53 or a part of it may be inclined from the rotation axis Lc to one side in the circumferential direction of the shaft 11. In other words, the inner wall surface 53a of the recessed groove 53 may be inclined helically from the rotation axis Lc at a predetermined angle. In this case, as the surgical bur 10 rotates, a flow along the shaft 11 will be generated in the perfusion fluid W.

As in the liquid flow generation parts 15, 15A, 15B, and 15C described above, the liquid flow generation part can create a flow in the perfusion fluid W with any protrusion or recess on the shaft 11 in the radial direction, and any shape that can generate a flow in the perfusion fluid W is acceptable.

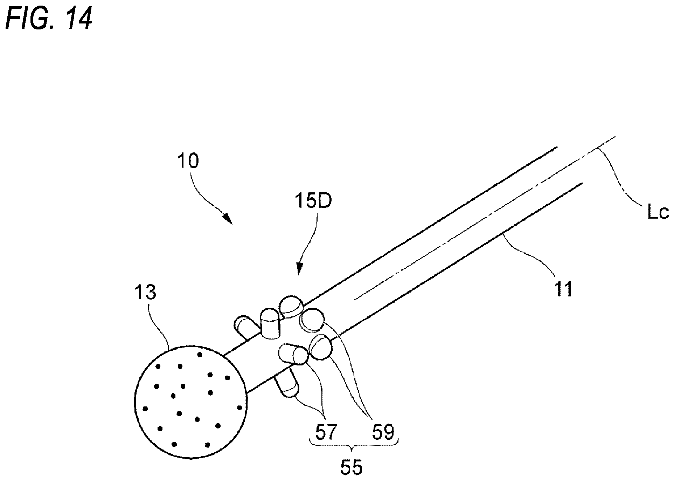

FIG. 14 is a perspective view showing the tip of the surgical bur 10 according to a third modification of the liquid flow generation part 15D. This liquid flow generation part 15D includes multiple rod-shaped bodies 55 protruding outward in the radial direction from the outer peripheral surface of the shaft 11. The rod-shaped bodies 55 include multiple tip-side rod-shaped bodies 57 with a longer protruding length, positioned closer to the cutting part 13, and multiple base-side rod-shaped bodies 59 with a shorter protruding length, positioned away from the cutting part 13. The rod-shaped bodies 55 are arranged at arbitrary positions along the circumference and preferably at multiple rotation symmetric positions centered on the rotation axis Lc. Additional rod-shaped bodies may be provided between the tip-side rod-shaped bodies 57 and the base-side rod-shaped bodies 59. As the surgical bur 10 rotates, the rod-shaped bodies 55 stir the perfusion fluid W, generating a flow in the perfusion fluid W.

The configurations related to the present invention is not limited to the embodiments described above and can be appropriately modified and improved. Other configurations such as materials, shapes, dimensions, numbers, forms, locations, etc., of the components described in the above embodiments are optional as long as the invention can be achieved and are not limited.

As described above, the present disclosure includes the following configurations:
(1) A surgical bur including a bar-shaped shaft and a cutting part provided at one axial end of the shaft, which rotates around the axis of the shaft,
   wherein the shaft is formed on the outer peripheral surface on the cutting part side in the axial direction and includes a liquid flow generation part having at least one of a convex part projecting outward in the radial direction or a concave part recessed inward in the radial direction.
   This surgical bur causes the liquid flow generated by the convex or concave liquid flow generation part formed on the shaft during surgery to remove perfusion fluid containing tissue fragments generated around the cutting part from the vicinity of the cutting position, thereby stabilizing the surgeon's field of view around the cutting position.
(2) The surgical bur according to (1) wherein the flow generation part including a spiral protrusion formed spirally around the outer circumference of the shaft centering on the axis, protruding outward from the outer peripheral surface of the shaft,
   wherein the surgical bur generates a flow in the perfusion fluid around the spiral protrusion by the feed motion of the spiral protrusion generated by rotating the shaft.
(3) The surgical bur according to (2), wherein the spiral protrusion has a lower protruding height in the radial direction closer to the cutting part.
   This surgical bur suppresses the obstruction of the surgeon's view near the cutting part by the spiral protrusion during surgery, making it easier to secure a wider field of view.
(4) The surgical bur according to (2), wherein the maximum outer diameter dimension of the spiral protrusion is smaller than the maximum outer diameter dimension of the cutting part.
   This surgical bur suppresses the obstruction of the surgeon's view by the spiral protrusion during surgery, making it easier for the surgeon to secure the view near the cutting part. Additionally, because the protrusion height of the spiral protrusion is lower than that of the cutting part, unnecessary tissue is less likely to be inadvertently damaged by the spiral protrusion.
(5) A surgical system including:
   the surgical bur described in any one of (1) to (4);
   a handpiece having a long tubular support that exposes the cutting part and the liquid flow generation part at the tip and rotationally supports the shaft;
   a rotation control device that rotationally drives the shaft of the surgical bur supported by the tubular support; and
   a perfusion fluid device that supplies perfusion fluid towards the cutting part and the liquid flow generation part of the surgical bur.
   This surgical system can ensure a good view of the condition during the cutting operation by rotating the cutting part and the liquid flow generation part in the liquid perfusion fluid at the affected area.
(6) The surgical system according to (5), further including:
   a sheath with a cylindrical tip inserted into the affected area, a tubular support of the handpiece inserted into the sheath; and
   an observation optical system obtaining an observational image of the affected area around the cutting part of the surgical bur.
   This surgical system allows cutting with the surgical bur through the sheath inserted into the affected area, and the observation optical system within the sheath can acquire the cutting condition.
(7) The surgical system according to (6), wherein the perfusion fluid device suctions and discharges the perfusion fluid in the sheath's cylinder.
   This surgical system can remove the perfusion fluid accumulating around the affected area by suctioning it in the sheath's cylinder.
(8) The surgical system according to (6), wherein the observation optical system includes an endoscopic device obtaining the observational image from the tip inserted into the affected area.
   This surgical system allows the surgeon to easily observe the cutting condition by the cutting part using the endoscopic device.
(9) A method for operating the surgical system described in (5), including:
   inserting the tip of the tubular support of the handpiece supporting the surgical bur into the affected area, supplying the perfusion fluid towards the cutting area by the surgical bur using the perfusion fluid device; and
   rotationally driving the surgical bur with the rotation control device while the cutting part and the liquid flow generation part are placed in the liquid perfusion fluid accumulated in the affected area, generating a flow of the perfusion fluid by the liquid flow generation part.
   This method for operating the surgical system allows suspended perfusion fluid near the cutting part to be replaced with new perfusion fluid, maintaining good visibility of the observation image around the cutting part.
(10) The method for operating the surgical system described in (9), wherein the liquid flow generation part generates a liquid flow along the shaft towards the liquid flow generation part with the rotation control device rotating the surgical bur, directing the perfusion fluid around the cutting part.
   This method for operating the surgical system removes the perfusion fluid containing tissue fragments cut by the cutting part from the vicinity of the cutting part by flowing along the shaft towards the liquid flow generation part, constantly filling the area around the cutting part with new perfusion fluid.
(11) The method for operating the surgical system described in (9), wherein the liquid flow generation part generates a liquid flow towards the opposite side of the shaft with the rotation control device rotating the surgical bur, directing the perfusion fluid around the cutting part.
   This method for operating the surgical system supplies new perfusion fluid to the cutting part, replacing the perfusion fluid containing tissue fragments cut by the cutting part from the vicinity of the cutting part, constantly filling the area around the cutting part with new perfusion fluid.

## Claims

1. A surgical bur comprising:
a shaft extending along an axis;
a cutting part provided on the shaft at one end in an axial direction of the shaft; and
a liquid flow generation part formed on an outer peripheral surface of the shaft at a position close to the cutting part, the liquid flow generation part having at least one of a convex part projecting outward in a radial direction of the shaft or a concave part recessed inward in the radial direction of the shaft and being configured to generate liquid flow when the shaft is rotated around the axis.

2. The surgical bur according to claim 1,
wherein the liquid flow generation part includes a spiral protrusion formed spirally around the outer peripheral surface of the shaft centering on the axis, and protruding outward from the outer peripheral surface of the shaft.

3. The surgical bur according to claim 2,
wherein the spiral protrusion has a lower protruding height in the radial direction toward a position closer to the cutting part.

4. The surgical bur according to claim 2,
wherein a maximum outer diameter dimension of the spiral protrusion is smaller than a maximum outer diameter dimension of the cutting part.

5. A surgical system comprising:
the surgical bur according to any one of claims 1 to 4,
a handpiece having a longitudinal tubular support that exposes the cutting part and the liquid flow generation part at the tip and rotationally supports the shaft;
a rotation control device that rotationally drives the shaft of the surgical bur supported by the tubular support; and
a perfusion fluid device that supplies perfusion fluid towards the cutting part and the liquid flow generation part of the surgical bur.

6. The surgical system according to claim 5, further comprising:
a sheath having a cylindrical shape to be inserted into an affected area and through which the longitudinal tubular support of the handpiece is inserted into; and
an observation optical system that allows a user to view an observational image of the affected area around the cutting part of the surgical bur.

7. The surgical system according to claim 6,
wherein the perfusion fluid device suctions and discharges the perfusion fluid in the sheath.

8. The surgical system according to claim 6,
wherein the observation optical system includes an endoscopic device that obtains the observational image from a tip end thereof inserted into the affected area.

9. A method for operating the surgical system according to claim 5, the method comprising:
supplying the perfusion fluid towards the cutting area by the surgical bur using the perfusion fluid device while inserting the tip of the longitudinal tubular support of the handpiece supporting the surgical bur into the affected area; and
rotationally driving the surgical bur with the rotation control device while the cutting part and the liquid flow generation part are placed in the liquid perfusion fluid accumulated in the affected area, generating a flow of the perfusion fluid by the liquid flow generation part.

10. The method according to claim 9,
wherein the liquid flow generation part generates a liquid flow directing the perfusion fluid around the cutting part along the shaft towards the liquid flow generation part with the rotation control device rotating the surgical bur.

11. The method according to claim 9,
wherein the liquid flow generation part generates a liquid flow directing the perfusion fluid around the cutting part towards a side further to the shaft with the rotation control device rotating the surgical bur.
